Europäisches Patentamt

European Patent Office

Office européen des brevets

(19)

(11) Veröffentlichungsnummer : **0 287 978 B1**

(12) **EUROPÄISCHE PATENTSCHRIFT**

(45) Veröffentlichungstag der Patentschrift :
26.06.91 Patentblatt 91/26

(51) Int. Cl.⁵ : **B01J 2/22, A61K 9/16**

(21) Anmeldenummer : **88106096.6**

(22) Anmeldetag : **16.04.88**

(54) **In Form eines Granulats vorliegendes Substanzgemisch zur Zubereitung von Dialyselösungen, Verfahren zu seiner Herstellung.**

(30) Priorität : **23.04.87 FR 8706339**

(43) Veröffentlichungstag der Anmeldung :
**26.10.88 Patentblatt 88/43**

(45) Bekanntmachung des Hinweises auf die
Patenterteilung :
**26.06.91 Patentblatt 91/26**

(84) Benannte Vertragsstaaten :
**DE ES FR GB IT SE**

(56) Entgegenhaltungen :
DE-A- 2 014 651
DE-A- 2 020 619
DE-A- 2 247 518
FR-A- 1 523 020
GB-A- 663 614

(73) Patentinhaber : **Fresenius AG**
**Gluckensteinweg 5**
**W-6380 Bad Homburg v.d.H. (DE)**

(72) Erfinder : **Weber, Daniel**
**La Clotte**
**F-47220 Astaffort (FR)**

(74) Vertreter : **Dr. Fuchs, Dr. Luderschmidt**
**Dipl.-Phys. Seids, Dr. Mehler Patentanwälte**
**Abraham-Lincoln-Strasse 7**
**W-6200 Wiesbaden (DE)**

## Beschreibung

Vorliegende Erfindung betrifft ein Substanzgemisch aus ionischen Komponenten zur Zubereitung von Dialyselösungen, das als Granulat ausgebildet ist, ein Verfahren zu dessen Herstellung sowie eine Vorrichtung zu deren Durchführung.

Der Erfindung liegt die Aufgabe zugrunde, ein Gemisch an sich bekannter ionischer Komponenten von Dialyselösungen in einer Applikationsform sowie ein Verfahren zu deren Herstellung zur Verfügung zu stellen, die stabil, gut lager-, transportier- und handhabbar sowie zur Zubereitung von Dialyselösungen und deren Konzentraten in hohem Maß geeignet ist.

Die Lösung dieser Aufgabe wird durch die in den kennzeichnenden Teilen der Patentansprüche vorgeschlagenen technischen Mittel erreicht.

Die Behandlung der chronischen Niereninsuffizienz durch außerkörperliche Zirkulierung des Patientenblutes zum Zweck seiner Reinigung erfordert, welche die benutzte Technik auch immer ist, die Handhabung von beträchtlichen Flüssigkeitsmengen.

Bei einem Dialysebetrieb wird das Blut des Patienten in einer künstlichen Niere gereinigt, in der eine Dialyselösung, oder ein Dialysat, umläuft, deren bzw. dessen elektrolytische Zusammensetzung mit derjenigen des Blutplasmas verwandt ist. Eine Dialyselösung umfaßt einen erhöhten Gehalt an Natrium, an Acetat oder an Bicarbonat (je nachdem, ob es sich um Acetat-Dialysat oder um ein Bicarbonat-Dialysat handelt), an Chloriden, und in geringeren Verhältnissen andere Ionen wie Calcium, Magnesium und Kalium. Im Laufe von drei Dialysebehandlungen pro Woche verbraucht jeder Patient 120 bis 200 l Dialysat pro Behandlung.

Diese beträchtlichen Volumina an Dialysat wurden bislang am Fuß des Patientenbettes mit Hilfe eines Apparates kontinuierlich hergestellt, der als Dialysegenerator bezeichnet wird, welcher die Auflösung einer konzentrierten Flüssigkeit im Wasser in Verhältnissen von 1 Volumen Konzentrat pro 34 Volumina Wasser gewährleistet. Für eine herkömmliche Acetat-Dialyse ist es angebracht, über 5 l Konzentrat zu verfügen, während für eine Bicarbonat-Dialyse es angebracht ist, über 2 Konzentrate, nämlich 5 l saures Konzentrat und 7 l Bicarbonat-Konzentrat zu verfügen, wobei diese beiden Elemente erst im letzten Moment aufgrund der Tatsache vermischt werden, daß sie nicht in Mischung unter einer stabilen Form aufbewahrt werden können.

Diese Technik stellt bei Bicarbonat-Konzentraten zahlreiche Probleme, insbesondere dasjenige der Aufbewahrung konzentrierter Flüssigkeiten, die unter strengen Temperaturbedingungen bewirkt werden muß und die nur während verhältnismäßig kurzer Zeiträume in der Größenordnung eines Monats gewährleistet werden kann. Überdies erfordern diese konzentrierten Flüssigkeiten die Handhabung beträchtlicher Mengen, da jede Dialyse, je nach dem Dialysetyp, zwischen 750 und 1.800 l Konzentrat pro Jahr verbraucht. Schließlich eignet sich die zur Herstellung einer Acetat-Dialyseflüssigkeit bestimmten herkömmlichen Dialysegeneratoren nicht für die Herstellung einer Bicarbonat-Dialyseflüssigkeit.

Diese unterschiedlichen Notwendigkeiten beschränken beträchtlich die Verbreitung der Bicarbonat-Dialyse, insbesondere die Durchführung dieses Dialysetyps am Wohnsitz des Patienten.

Auch sind Zentralen zur Herstellung von Dialyseflüssigkeiten vorhanden, welche zur gleichzeitigen Versorgung mehrerer Patienten bestimmt sind, die nach dem gleichen Prinzip arbeiten.

Es wurde schon vorgeschlagen, Konzentrate in Form von Pulver herzustellen, das vor der Behandlung in Wasser aufzulösen ist. Das Konzentrat wird in der Regel durch den Apotheker des Dialysezentrums, ausgehend von Lagerprodukten in Form von Pulvern, hergestellt. Diese Produkte werden abgewogen und sodann in einem mit einem Rührer versehenen Behälter aufgelöst. Nach einer ausreichenden Rührzeit werden die durch das Arzneimittelbuch vorgesehenen Kontrollen durchgeführt. Nach Filtration wird das Produkt einem zweiten Ionogramm unterworfen, wonach das Präparat zu einem Lagerbehälter gefördert wird. Die Kompliziertheit der Durchführung dieses Verfahrens begrenzt seine Anwendung auf Dialysezentren, die über eine bedeutende Infrastruktur verfügen, aufgrund der Tatsache, daß das Handhaben und Abwiegen der Produkte heikel sind, und daß es notwendig sein kann, infolge von Wasseraufnahme bestimmter Produkte Korrekturen anzubringen.

Demgegenüber bieten die erfindungsgemäß vorgeschlagenen Granulate, die ausschließlich aus ionischen, für die Dialyse erforderlichen Substanzen bestehen, und das Verfahren zu ihrer Herstellung folgende wesentlichen Vorteile :

Die Herstellung von Granalien einer regelmäßigen Konsistenz liefert eine gleichförmige Mischung, wobei jedes Risiko einer Klassifikation im Laufe des Transports oder der Handhabung vermieden wird, wie es bei heterogenen Teilchen der Fall ist.

Die granulierten Materialien sind leichter und widerstehen der Bildung von Rundungen (voûtes) und der Agglutinierung (das Granulat ist nicht hygroskopisch) ; schnellere Durchsätze und ein regelmäßigeres Auffüllen können so erhalten werden.

Schließlich absorbieren die granulierten Materialien die Flüssigkeiten schneller als die Mehrzahl von Pulvern (die Granulierung erlaubt, die Kontaktoberfläche zwischen dem Produkt und dem Lösungsmittel zu vergrößern). Infolgedessen dispergieren und lösen sich diese granulierten Materialien leichter und schneller.

Die vorteilhaften Eigenschaften zeigen insbesondere Granalien gemäß der Erfindung mit einer Teilchengröße von 0,5-3 mm. Die physikalisch-chemischen Eigenschaften des Granulats und seine Konditionierung erlauben eine einfache aber wirksame Anwendung zur Herstellung in situ des Konzentrats zur Hämodialyse : das Zentrum vermeidet so die mit einer Untersuchung der Erstsubstanzen und allen anderen Schwierigkeiten der Herstellung verbundenen Probleme (Handhabung (evtl. Vermahlen), Abwiegen,...).

Ein anderer gewichtiger Vorteil stellt die Wirtschaftlichkeit dar : tatsächlich ist der Gestehungspreis eines Liters mit dem Granulat hergestellten Konzentrats (unter Berücksichtigung der für diese Herstellung in situ erforderlichen Installationen) vorteilhafter als derjenige bekannter flüssiger Konzentrate.

Infolgedessen ermöglicht das Granulat, Dialysenzentren mit Produkten zur Herstellung von Konzentraten an Ort und Stelle zu versorgen, wobei ihnen die Untersuchung der Rohstoffe und alle Schwierigkeiten bei der Herstellung erspart bleiben (Schwierigkeiten der Handhabung, des Wiegens, der Dosierungen und Kontrollen u.s.w.),
eine Ersparnis für diese Zentren und die hinsichtlich des Exports angetroffenen Erfordernisse zu erfüllen (geringeres Volumen, nichthygroskopische Produkte, die sich gut aufbewahren lassen).

Das Verfahren zur Herstellung des Granulats besteht darin, daß man die verschiedenen, in Pulverform vorliegenden Komponenten in den gewünschten Verhältnissen, vor oder nach einer Zerkleinerung der Teilchen auf die gewünschte Größe, vermischt,
die Teilchen auf trockenem Wege unter Bildung von Preßlingen verdichtet, und
die Preßlinge zu Granalien vermahlt.

Vorteilhafterweise wird das Verpressen der Teilchen durchgeführt, indem man ein Gemisch der fein vermahlenen Teilchen unter Druck zwischen zwei Walzen mit parallelen Achsen hindurchführt, die sich in einander gegenläufigem Sinn umdrehen.

Gemäß einer ersten Durchführungsform besteht das Verfahren darin, daß zuerst das Vermischen der in Pulverform vorliegenden Bestandteile ausgeführt wird, und zwar in den gewünschten Verhältnissen, und danach das Zerkleinern der Teilchen des Gemisches, um sie auf eine geringere Größe zu bringen.

Gemäß einer anderen Ausführungsform besteht das Verfahren darin, daß man zuerst das Zerkleinern der Teilchen, welche jeden Bestandteil bilden, durchführt, um sie auf die gewünschte Größe zu bringen, und danach das Vermischen der verschiedenen Bestandteile in den gewünschten Verhältnissen.

Vorteilhafterweise besteht das Verfahren im Zerkleinern der die verschiedenen Bestandteile bildenden Teilchen, um Teilchen mit einer Größe von weniger als 200 µm zu erhalten, und im Verdichten des Gemisches in Form von Preßlingen, welche so verdichtet sind, daß man Granalien mit einer Größe von 0,5-3 mm erhält. Gemäß einer möglichen Ausführungsform besteht dieses Verfahren darin, daß man, ausgehend von Komponenten, welche im Gemisch in sehr unterschiedlichen Anteilen vorliegen sollen, ein Vormischen der Komponenten durchführt, welche in den geringsten Anteilen vorliegen, wobei man dieses Vorgemisch selbst danach den Komponenten zumischt, welche in den bedeutenderen Anteilen vorliegen. Dies ermöglicht die im Rahmen der eigentlichen Granalienherstellung erforderlichen Arbeitsgänge sowie das Volumen der Einrichtung zu begrenzen.

Vorteilhafterweise besteht das Verfahren darin, daß man ein doppeltes Sieben der vermahlenen Teilchen durchführt und einerseits die Teilchen mit einer Größe unterhalb der zugelassenen Minimalgröße und andererseits die Teilchen mit einer Größe oberhalb der zugelassenen Maximalgröße zur Verdichtung rückführt.

Das Verfahren ist unter wirtschaftlichen Gesichtspunkten sehr vorteilhaft, weil es erlaubt, ein Granulat gemäß der Erfindung unter Herstellungskosten zu erhalten, welche sehr wesentlich unterhalb denjenigen anderer Verfahren liegen, wie z.B. der Lyophilisierung, Versprühnebelung oder Granulierung auf dem Feuchtweg, welche einen beträchtlichen Energieverbrauch erfordern.

Aus der DE-OS 20 20 619 wurde ein Verfahren zur Herstellung einer Trägersubstanz für Tablettenbestandteile, die unmittelbar zu Tabletten verpreßbar sein sollen, bekannt, bei dem man ein Gemisch der Tablettenbestandteile zusammen mit einem Preßhilfsmittel mittels einer Preßeinrichtung, z.B. mittels Preßwalzen, zu einer festen, nicht bröckelnden Tafel verformt, und die Tafel mit einer Zerkleinerungsvorrichtung, z.B. mit "Fitsmills", in Teilchen aufbricht, wobei man anschließend zwecks Erhalts eines Granulats des gewünschten Korngrößenbereichs ein Sieben durchführen kann.

Die DE-OS 2 014 651 beschreibt ein Verfahren zur Herstellung eines gasabgebenden, aufbrausenden Reinigungsmittels für Zahnprothesen in Granulatform, bei dem eine Mischung der Reinigungskomponenten in Pulverform komprimiert, entlüftet und sodann, z.B. mittels zwei, in einander entgegengesetztem Drehungssinn rotierenden Druckwalzen, zu einer dichten Materialbahn extrudiert wird, die zu einem Granulat mit annähernd der gewünschten Teilchengröße zerkleinert wird, von dem sodann die zu groben und zu feinen Teilchen abgetrennt werden, welche in die Ausgangsmischung zurückgeführt werden können.

Die beiden Druckschriften zugrundeliegenden Aufgabenstellungen gehen von einem unterschiedlichen technologischen Standort aus und konnten nichts zur Lösung der vorliegender Erfindung zugrundeliegenden Aufgabe beitragen.

Gleiches gilt für folgende Druckschriften, sei es, daß man sie für sich allein oder in Zusammenschau betrachtet.

Aus der GB-PS 663,614 ist ein Verfahren zur Agglomerierung von pulverförmigen Teilchen von u.a. Mineralsalzen, insbesondere Ammoniumchlorid, bekannt, bei dem die unmittelbar vor ihrer Agglomeration auf 90°C bis 100°C erhitzten Teilchen in angefeuchtetem Zustand unter verhältnismäßig geringem Druck, vorzugsweise durch einen Luppenhammer ("ball press" 15), zu tetraedrischen Kuchen mit einem Gewicht von 20 g agglomeriert werden.

Schließlich wurde aus der FR-PS 1.523.020 ein Verfahren zur Agglomeration von Stoffen, die einer metallurgischen Behandlung unterworfen werden sollen, bekannt, wobei die Stoffe zwischen zwei Walzen unter Erhitzen gewalzt, und die erhaltenen Granulate oder Preßlinge danach in einem zweiten Arbeitsgang zerkleinert werden, um sie für eine nachfolgende metallurgische Behandlung geeignet zu machen. Es wird betont, daß hierbei der Form des Agglomerats kein Vorzug gegeben wird, da dieses anschließend ohnehin zerkleinert wird, und der Zweck des Walzens nicht die Herstellung eines Produkts mit guter mechanischer Widerstandsfähigkeit ist.

Zur Durchführung des erfindungsgemäßen Verfahrens ist eine Vorrichtung geeignet, welche zwei Walzen mit parallelen Achsen, die gegenläufig angetrieben sind, umfaßt, zwischen denen das Komponentengemisch unter Druck durchgeführt wird, und die dessen Verdichtung in Form von Preßlingen bewirken, wobei die Preßlinge auf eine Mahlvorrichtung fallen, welche die Bildung von Granalien gewährleistet, die auf zwei übereinander horizontal angeordnete Siebe fallen, wobei das erste Sieb die Granalien mit einer Größe oberhalb der zugelassenen Maximalgröße, und das zweite Sieb die Granalien mit einer Größe unterhalb der zugelassenen Minimalgröße durchgehen lassen ; diese beiden Arten von Granalien werden zu einem neuen Durchgang zwischen den Walzen rückgeführt, während die durch das zweite Sieb zurückgehaltenen Granalien zu einem Lagerungssilo gefördert werden.

Die Verdichtungswalzen können horizontale oder vertikale Achsen aufweisen.

Gemäß einer ersten Ausführungsform umfaßt diese Vorrichtung mehrere Lagerungssilos für Bestandteile oder Vormischungen von Bestandteilen, unterhalb eines jeden derselben ein Fülltrichter angeordnet ist, der in einen Sammler mündet, wobei die Teilchen in einen Mischer fallen, danach in eine Pulverisierungsvorrichtung, welche deren Größe vermindert, und wobei die Teilchen sodann durch einen Löffelaufzug aufgenommen werden, der sie in einen Trichter führt, von wo sie zwischen die beiden Walzen transportiert werden.

Zweckmäßigerweise sind die Trichter zur Dosierung der verschiedenen Komponenten oder Vorgemische von Komponenten solche mit einer Gewichtsdosierung.

Überdies wird der Durchgang von dem oben am Aufzug gelegenen Trichter zu den Verdichtungswalzen durch eine erste Transportschraube mit einer horizontalen Achse bewerkstelligt, deren Mantel in den Mantel einer zweiten Schraube mit vertikaler Achse mündet, welche die Teilchen unter Druck zwischen die beiden Walzen führt, die auf einem Abstand gehalten werden, der voneinander unter der Einwirkung elastischer Mittel bestimmt wird.

Diese Vorrichtung ist, oberhalb der Lagerungssilos für die Komponenten des Gemischs, mit einem Trichter zur Beschickung der verschiedenen Komponenten unabhängig voneinander ausgestattet, wobei jede zum entsprechenden Lagerungssilo durch eine pneumatische Transportvorrichtung gefördert wird, welche mit Dreiwegschiebern versehen ist, von denen jeder die Transportvorrichtung mit einem Silo in Verbindung bringen kann.

Die Schieber zur Verbindung der pneumatischen Transportvorrichtung mit den Silos sind von der Ablesung eines Codes ab auf Abstand gesteuert, der auf jeder Verpackung der Komponenten angebracht ist, die in den Beschickungstrichter geschüttet werden.

Die pneumatische Transportvorrichtung ist mit einem Schieber zum In-Verbindung-Bringen mit einem Trichter für die Mischung versehen, der ggf. dazu bestimmt ist, das Vorgemisch der in geringen Mengen vorliegenden Komponenten durchzuführen.

Bei einer anderen Ausführungsform kann die Vorrichtung zur Herstellung der erfindungsgemäßen Granulate auch zwei parallel angeordnete Mischer umfassen, von denen einjeder zur Aufnahme der unterschiedlichen pulverförmigen Komponenten in den erforderlichen Anteilen bestimmt ist, wobei der eine und der andere dieser Mischer alternativ eine Pulverisierungsvorrichtung speist, welche die Teilchengröße herabsetzt, und die oberhalb der Verdichtungswalzen angebracht ist.

Vorteilhafterweise umfaßt die Vorrichtung einerseits eine Einrichtung zur Trocknung der Luft der Produktionsanlage und andererseits eine Einrichtung zur Trocknung der Teilchen der unterschiedlichen Komponenten durch Temperaturerhöhung, beispielsweise auf einem Fluidbett.

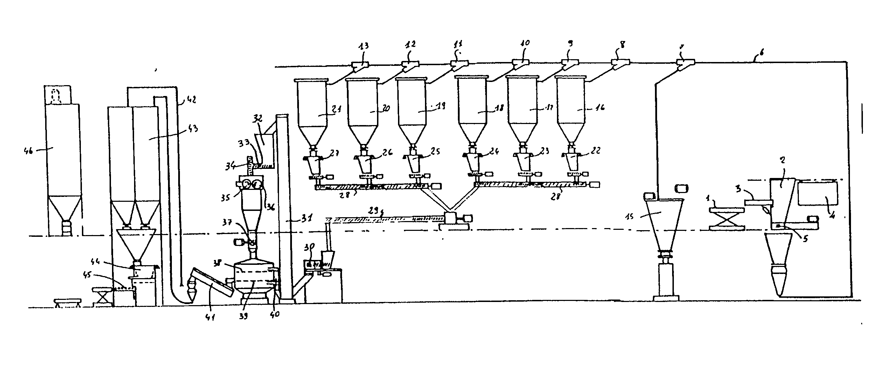

Auf alle Fälle wird die Erfindung mit Hilfe der folgenden Beschreibung unter Bezugnahme auf die beigefügte schematische Zeichnung gut verstanden, deren einzige Abbildung schematisch eine geeignete Vorrichtung zur Herstellung des Granulats gemäß der Erfindung darstellt.

Die in der Zeichnung dargestellte Vorrichtung umfaßt einen Trichter (2) zur Beschickung der verschiedenen Komponenten des Granulats, wobei die unterschiedlichen Komponenten unabhängig voneinander, beispielsweise in Säcken aus Polyethylen, herbeigebracht werden. Jeder Sack wird auf eine Bühne (3) gestellt, was nach Aufstellung einer Schutzhaube (4) die Entleerung seines Inhaltes ermöglicht. Nachdem man es einer Entklumpung mit Hilfe einer am Boden aus Trichters (2) angeordneten Vorrichtung unterwarf, wird das Produkt mittels einer pneumatischen Transportvorrichtung (6) aufgenommen. Auf der Leitung der pneumatischen Transportvorrichtung sind 7 in Reihe angeordnete Dreiwegschieber (7 bis 13) angebracht.

Der erste Schieber (7) ermöglicht den Durchgang einer Komponente sowohl zum zweiten Schieber (8) als auch zu einem Mischtrichter (15). Dieser Mischtrichter wird zur Durchführung einer speziellen Produktion bestimmter Produkte benutzt, welche sich in geringen Anteilen in den Granalien befinden.

Der Schieber (8) ermöglicht den Durchgang der Komponenten zu einem Silo (16) oder zu dem Schieber (9), der Schieber (9) ermöglicht den Durchgang der Komponenten zu einem Silo (17) oder zu dem Schieber (10), und der Schieber (10) erlaubt den Durchgang der Komponenten zu einem Silo (18) u.s.w..

Beispielsweise kann der Silo (16) zur Lagerung von Calciumsalzen, Silo (17) zur Lagerung von Magnesiumsalzen, Silo (18) zur Lagerung von Kaliumsalzen, Silo (19) zur Lagerung von Natriumchlorid, Silo (20) zur Lagerung von Natriumacetat, und Silo (21) schließlich zur Lagerung von Natriumbicarbonat dienen.

In Funktion der Codes, die auf den, die verschiedenen Komponenten enthaltenden Säcken angebracht sind, werden die Schieber automatisch betätigt, um die pneumatische Förderung jedes Produkts zu dem Silo, der ihm zugeordnet ist, zu gewährleisten, z.B. von Natriumchlorid zum Silo (19).

Unterhalb eines jeden Silos (16-21) ist ein Dosierungstrichter (22-27) angeordnet, wodurch ein Durchtritt einjeder der Komponenten in den vorbestimmten Anteilen in ein Tunnel gewährleistet ist, das Transportbänder 28 enthält, welche die verschiedenen Komponenten zu einem Zentralpunkt führen. Von diesem Zentralpunkt werden die Komponenten durch Schwerkraft zu einem Schraubenmischer (29) abgeführt. Nach dem Vermischen werden die Teilchen einer Größe zwischen 0,8 und 1 mm durch Schwerkraft zu einer Pulverisierungsvorrichtung (30) transportiert, die einen Messerzerkleinerer umfaßt, der die Teilchen auf eine Größenordnung von 200 µm bringt. Am Ausgang der Pulverisierungsvorrichtung werden die Teilchen durch einen Aufzug (31) aufgenommen, der sie zu einem Trichter (32) bringt. Am Boden dieses Trichters (32) ist eine Schraube (33) angebracht, deren Mantel in den Mantel einer endlosen Schraube (34) mit vertikaler Achse mündet, welche die Teilchen zwischen zwei Walzen (35 und 36) drückt, die in gegeneinanderläufigem Sinn rotieren gelassen werden, derart, daß die Bildung von Preßlingen gewährleistet ist und sie nach unten fortbewegt werden. Zumindest eine der beiden Walzen ist gegenüber der anderen seitlich verschiebbar · angebracht, wobei diese Montage elastisch ist.

Die durch Kompression im trockenen Zustand zwischen den beiden Walzen gebildeten Preßlinge laufen zu einer Mahlvorrichtung (37), welche die Bildung von Granalien gewährleistet. Diese Granalien fallen auf zwei übereinander angeordnete horizontale Siebe (38 und 39), von denen das obere (38) die Teilchen mit einer Größe von weniger als 3 mm, und das untere (39) die Teilchen mit einer Größe von weniger als 0,5 mm durchgehen läßt. Die vom ersten Sieb zurückgehaltenen und die das zweite Sieb durchlaufenden Teilchen werden durch einen Stutzen (40) aufgenommen, der sie zum Aufzug (31) rückführt.

Die als verwendbar erachteten Teilchen einer Größe zwischen 0,5 und 3 mm werden durch eine Transportvorrichtung (41) zu einem Lagerungssilo (43) gefördert.

Unterhalb des Lagerungssilos sind Vorrichtungen zum Abwiegen (44) und Abfüllen (45) angebracht. Diese Vorrichtung umfaßt schließlich eine Gruppe von Entstaubungsvorrichtungen (46).

## Ansprüche

1. Feststoffgemisch, bestehend aus den ionischen Komponenten einer Dialyselösung, dadurch gekennzeichnet, daß es als Granulat vorliegt.

2. Gemisch gemäß Anspruch 1, dadurch gekennzeichnet, daß die Granulatteilchen eine Größe von 0,5 bis 3 mm aufweisen.

3. Verfahren zur Herstellung des Granulats gemäß einem der Ansprüche 1 und 2, dadurch gekennzeichnet, daß man die verschiedenen, in Pulverform vorliegenden Komponenten in den gewünschten Verhältnissen, vor oder nach einer Zerkleinerung der Teilchen auf die gewünschte Größe, vermischt, die Teilchen auf trockenem Wege unter Bildung von Preßlingen verdichtet, und die Preßlinge zu Granalien vermahlt.

4. Verfahren gemäß Anspruch 3, dadurch gekennzeichnet, daß man das Verdichten der Teilchen durch Durchführen eines Gemischs von fein zerkleinerten Teilchen unter Druck zwischen zwei Walzen mit parallelen Achsen durchführt, welche in einander gegenläufigem Umdrehungssinn angetrieben werden.

5. Verfahren gemäß einem der Ansprüche 3 und 4, dadurch gekennzeichnet, daß man zuerst das Vermischen der verschiedenen, in Pulverform vorliegende Komponenten in den gewünschten Verhältnissen, und danach das Zerkleinern der Teilchen des Gemischs durchführt, um sie auf eine geringere Größe zu bringen.

6. Verfahren gemäß einem der Ansprüche 3 und 4, dadurch gekennzeichnet, daß man zuerst das Zerkleinern der jede Komponente bildenden Teilchen durchführt, um sie auf die gewünschte Größe zubringen, und danach das Vermischen der verschiedenen Komponenten in den gewünschten Verhältnissen.

7. Verfahren gemäß einem der Ansprüche 3 bis 6, dadurch dadurch gekennzeichnet, daß man die die verschiedenen Komponenten bildenden Teilchen zu Teilchen mit einer Größe von weniger als 200 μm zerkleinert.

8. Verfahren gemäß einem der Ansprüche 3 bis 7, dadurch gekennzeichnet, daß man ausgehend von Komponenten, die im Gemisch in sehr unterschiedlichen Verhältnissen vorliegen sollen, ein Vormischender in den geringsten Anteilen vorliegenden Komponenten durchführt, wonach dieses Vorgemisch sodann selbst mit den Komponenten vermischt wird, welche in den beträchtlicheren Anteilen vorliegen.

9. Verfahren gemäß einem der Ansprüche 3 bis 8, dadurch gekennzeichnet, daß man ein zweifaches Sieben der vermahlenen Teilchen durchführt und daß man einerseits die Teilchen mit einer Größe unterhalb der zugelassenen Minimalgröße und andererseits die Teilchen mit einer Größe oberhalb der zugelassenen Maximalgröße zur Verdichtung rückführt.

## Claims

1. Solid mixture of ionic components of a dialysis solution, characterized in that it is present as granules.

2. Mixture according to claim 1, characterized in that the granules have a size of between 0,5 and 3 mm.

3. A process for manufacturing the granules according to one of claims 1 and 2, characterized in that
the various powdered components are mixed in the desired ratios before or after reduction of the particles to the desired size, that the particles are dry pressed to form briquettes and that the briquettes are ground to form granules.

4. The process according to claim 3, characterized in that the compression of the particles is carried out by feeding a mixture of finely divided particles under pressure through a pair of rollers with mutually parallel axes, running in mutually opposed directions.

5. The process according to one of claims 3 and 4, characterized in that first the various powdered components of the mixture are mixed in the desired ratios and that thereafter the particles of the mixture are reduced in size to bring them to a desired smaller size.

6. The method according to one of claims 3 and 4, characterized in that first the particles forming each component are reduced in size to the desired size and that thereafter the components are mixed in the desired mutual ratios.

7. The process according to one of claims 3 to 6, characterized in that the particles forming the components are reduced to particles having a size less than 200 μm.

8. The method according to one of claims 3 to 7, characterized in that proceeding from components which in the mixture shall be present in exceedingly different mutual ratios a premix is formed of those components of the smallest ratio and that then the premix itself is mixed with those components having the larger ratio.

9. The process according to one of claims 3 to 8, characterized in that a double sieving of the ground particles is carried out and that on the one hand the particles having a size less than the minimal size and on the other hand the particles having a size greater than the maximum size are recycled to the compression arrangement

## Revendications

1. Mélange de matières solides constitué des composants ioniques d'une solution de dialyse, caractérisé en ce qu'il se présente sous la forme de granulés.

2. Mélange selon la revendication 1, caractérisé en ce que les particules des granulés ont une grosseur comprise entre 0,5 et 3 mm.

3. Procédé de fabrication des granulés selon la revendication 1 ou 2, caractérisé en ce que
les différents composants, sous forme pulvérulente, sont mélangés dans les proportions souhaitées, avant ou après une fragmentation des particules à la grosseur souhaitée, les particules sont compactées par voie sèche pour former des comprimés et les comprimés sont broyés pour donner de la grenaille.

4. Procédé selon la revendication 3, caractérisé en ce que le compactage des particules s'effectue par passage d'un mélange de particules finement broyées, sous pression, entre deux cylindres d'axes parallèles qui sont entraînés dans un sens de rotation contraire l'un par rapport à l'autre.

5. Procédé selon la revendication 3 ou 4, caractérisé en ce qu'il est procédé tout d'abord au mélange des différents composants, sous forme pulvérulente, dans les proportions souhaitées puis à la fragmentation des particules du mélange, afin de les amener à des dimensions réduites.

6. Procédé selon la revendication 3 ou 4, caractérisé en ce qu'il est procédé tout d'abord à la fragmentation des particules formant chaque composant, afin de les amener aux dimensions souhaitées puis au mélange des différents composants, dans les proportions souhaitées.

7. Procédé selon l'une des revendications 3 à 6, caractérisé en ce que les particules formant les différents composants sont fragmentées en particules d'une grosseur inférieure à 200 µm.

8. Procédé selon l'une des revendications 3 à 7, caractérisé en ce que, partant de composants qui dans le mélange doivent avoir des proportions très différentes, les composants aux proportions les plus faibles sont préalablement mélangés, après quoi ils sont eux-mêmes mélangés aux composants se trouvant dans des proportions plus importantes.

9. Procédé selon l'une des revendications 3 à 8, caractérisé en ce qu'il est procédé à un double tamisage des particules broyées et en ce que d'une part les particules inférieures à la grosseur minimale admise et d'autre part les particules supérieures à la grosseur maximale admise sont renvoyées au compactage.